# EUROPEAN PATENT APPLICATION

(11) **EP 4 480 959 A1**
(43) Date of publication of application: **25.12.2024**
(21) Application number: 23721822.7
(22) Date of filing: 14.02.2023
(51) Int. Cl.: C07K 14/245, C12N 15/70, C12P 13/22

(54) **SIGMA 38 NOVEL VARIANT AND METHOD FOR PRODUCING L-AROMATIC AMINO ACID USING SAME**

(30) Priority: 16.02.2022 KR 20220020011
(71) Applicant: Daesang Corporation, Seoul 02586 (KR)
(72) Inventor: KIM, Hyun Young, Yongin-si Gyeonggi-do 17095 (KR); YANG, Cheol Min, Incheon 21035 (KR); HAN, Jong Yun, Yongin-si Gyeonggi-do 17048 (KR); JO, Young Il, Seoul 04995 (KR)
(74) Representative: Cabinet Nony
(86) International application number: PCT/KR2023/002095
(87) International publication number: WO 2023/158174

(57) **Abstract**

The present invention relates to a novel sigma 38 variant and a method of producing an L-aromatic amino acid using the same. The sigma 38 variant is obtained by substituting one or more amino acids in the amino acid sequence constituting sigma 38 to change protein activity, and a recombinant microorganism comprising the sigma 38 variant is capable of efficiently producing an L-aromatic amino acid.

## Description

### Technical Field

The present invention relates to a novel sigma 38 variant and a method of producing an L-aromatic amino acid using the same.

### Background Art

Amino acids are classified into hydrophobic, hydrophilic, basic and acidic amino acids, depending on the nature of their side chains, and among them, amino acids having a benzene ring are called aromatic amino acids. Aromatic amino acids include phenylalanine, tyrosine, and tryptophan, and phenylalanine and tryptophan are essential amino acids that are not synthesized *in vivo* and correspond to high value-added industries that form a global market worth $300 billion annually.

For the production of aromatic amino acids, either naturally occurring wild-type strains or mutant strains modified to have an increased ability to produce amino acids may be used. In recent years, in order to improve the efficiency of production of aromatic amino acids, there has been development of a variety of recombinant strains or mutant strains having excellent L-aromatic amino acid productivity by applying genetic recombination technology to microorganisms such as *Escherichia coli* and *Corynebacterium,* which are widely used in the production of useful substances such as L-amino acids, and methods of producing L-aromatic amino acids using the same. In particular, there have been attempts to increase the production of L-aromatic amino acids by inducing mutations in genes such as enzymes, transcription factors and transport proteins, which are involved in the biosynthetic pathways of the L-aromatic amino acids, or promoters that regulate the expression of these genes. However, there are dozens of types of proteins such as enzymes, transcription factors and transport proteins, which are involved directly or indirectly in the production of L-aromatic amino acids, and thus there is still a need for a lot of studies on whether changes in the activities of these proteins lead to an increase in L-aromatic amino acid productivity.

### [Prior Art Documents]

### [Patent Documents]

Korean Patent No. 10-1830002

### DISCLOSURE

### Technical Problem

An object of the present invention is to provide a novel sigma 38 variant.

Another object of the present invention is to provide a polynucleotide encoding the variant.

Still another object of the present invention is to provide a transformant comprising the variant or the polynucleotide.

Yet another object of the present invention is to provide a method of producing an L-aromatic amino acid using the transformant.

### Technical Solution

One aspect of the present invention provides a sigma 38 variant composed of the amino acid sequence of SEQ ID NO: 1 in which glutamine at position 37 in the amino acid sequence of SEQ ID NO: 3 is substituted with leucine.

"Sigma 38" used in the present invention is a type of sigma factor, a protein that binds to RNA polymerase, and functions to initiate transcription by recognizing and binding to the promoter of a gene. The sigma 38 may be a gene encoding sigma 38 or a sequence substantially identical thereto. As used herein, the term "substantially identical" means that, when analyzed by aligning each gene sequence, that is, a base sequence or nucleotide sequence, and any other nucleotide sequence for maximum correspondence, the other nucleotide sequence has a sequence homology of at least 70%, at least 80%, at least 90%, or at least 98% to each nucleotide sequence.

Sigma 38 in the present invention is encoded by the rpoS gene and comprises the amino acid sequence of SEQ ID NO: 3.

According to one embodiment of the present invention, the amino acid sequence of SEQ ID NO: 3 may be derived from wild-type *E. coli.*

As used herein, the term "variant" refers to a polypeptide which is obtained by conservative substitution and/or modification of one or more amino acids at the N-terminus, C-terminus of and/or within the amino acid sequence of a specific protein and has an amino acid sequence different from that of the protein before mutation, but retains functions or properties of the protein. As used herein, the term "conservative substitution" means replacing one amino acid with another amino acid having similar structural and/or chemical properties. The conservative substitution may have little or no impact on the activity of the resulting protein or polypeptide. In addition, some variants include those in which one or more portions, such as an N-terminal leader sequence or transmembrane domain, have been removed. Other variants include those in which a portion has been removed from the N- and/or C-terminus of a mature protein. The ability of the variant may be increased, unchanged, or reduced compared to that of the polypeptide before mutation. In the present invention, the term "variant" may be used interchangeably with terms such as mutant, modification, variant polypeptide, modified protein, mutation, and the like.

The variant in the present invention may be a sigma 38 variant composed of the amino acid sequence of SEQ ID NO: 1 in which the amino acid glutamine at position 37 in the amino acid sequence of SEQ ID NO: 3 is substituted with leucine.

Another aspect of the present invention provides a polynucleotide encoding the sigma 38 variant.

As used herein, the term "polynucleotide" refers to a DNA or RNA strand having a certain length or more, which is a long-chain polymer of nucleotides formed by linking nucleotide monomers via covalent bonds. More specifically, the term "polynucleotide" refers to a polynucleotide fragment encoding the variant.

The polynucleotide may comprise a nucleotide sequence encoding the amino acid sequence of SEQ ID NO: 1.

According to one embodiment of the present invention, the polynucleotide may comprise the nucleotide sequence represented by SEQ ID NO: 2.

Another aspect of the present invention provides a vector comprising a polynucleotide encoding the sigma 38 variant.

Still another aspect of the present invention provides a transformant comprising the sigma 38 variant or the polynucleotide.

As used herein, the term "vector" refers to any type of nucleic acid sequence transfer structure that is used as a means for transferring and expressing a gene of interest in a host cell. Unless otherwise specified, the term "vector" may mean one allowing the nucleic acid sequence contained therein to be expressed after insertion into the host cell genome and/or one allowing the nucleic acid sequence to be expressed independently. This vector comprises essential regulatory elements operably linked so that an inserted gene can be expressed. As used herein, the term "operably linked" means that a gene of interest and sequences for regulating expression of the gene are functionally linked together in a manner enabling gene expression, and the "regulatory elements" include a promoter for initiating transcription, any operator sequence for regulating transcription, a sequence encoding suitable mRNA ribosome binding sites, and a sequence for regulating termination of transcription and translation.

The vector that is used in the present invention is not particularly limited as long as it may replicate in a host cell, and any vector known in the art may be used. Examples of the vector include a natural or recombinant plasmid, cosmid, virus and bacteriophage. Examples of a phage vector or cosmid vector include, but are not limited to, pWE15, M13, λMBL3, λMBL4, λIXII, λASHII, λAPII, λt10, λt11, Charon4A, and Charon21A, and examples of a plasmid vector include, but are not limited to, pBR series, pUC series, pBluescriptII series, pGEM series, pTZ series, pCL series, and pET series.

The vector may typically be constructed as a vector for cloning or as a vector for expression. The vector for expression may be a conventional vector that is used in the art to express a foreign gene or protein in a plant, animal, or microorganism, and may be constructed through various methods known in the art.

The recombinant vector may be constructed to use a prokaryotic or eukaryotic cell as a host. For example, when the vector used is an expression vector and uses a prokaryotic cell as a host, the vector generally comprises a strong promoter capable of promoting transcription (e.g., pLλ promoter, CMV promoter, trp promoter, lac promoter, tac promoter, T7 promoter, etc.), a ribosome binding site for initiation of translation, and a transcription/translation termination sequence. When a eukaryotic cell is used as a host, the vector comprises a replication origin operating in the eukaryotic cell, such as an f1 replication origin, an SV40 replication origin, a pMB1 replication origin, an adeno replication origin, an AAV replication origin, or a BBV replication origin, without being limited thereto. In addition, the recombinant vector may comprise a promoter derived from the genome of a mammalian cell (e.g., metallothionein promoter) or a promoter derived from a mammalian virus (e.g., adenovirus late promoter, vaccinia virus 7.5K promoter, SV40 promoter, cytomegalovirus promoter, HSV-tk promoter, etc.), and generally has a polyadenylation sequence as a transcription termination sequence.

The recombinant vector may comprise a selection marker. The selection marker serves to select a transformant (host cell) transformed with the vector, and since only cells expressing the selection marker can survive in the medium treated with the selection marker, it is possible to select transformed cells. Representative examples of the selectable marker include, but are not limited to, kanamycin, streptomycin, and chloramphenicol.

The transformant may be produced by inserting the recombinant vector into a host cell, and the transformant may be obtained by introducing the recombinant vector into an appropriate host cell. The host cell is a cell capable of stably and continuously cloning or expressing the expression vector, and any host cell known in the art may be used.

When the vector is transformed into prokaryotic cells to generate recombinant microorganisms, examples of host cells that may be used include, but are not limited to, E. coli sp. strains such as *E. coli* JM109, *E. coli* BL21, *E. coli* RR1, *E. coli* LE392, *E*. *coli* B, *E. coli* X 1776, *E. coli* W3110, and *E. coli* XL1-Blue, *Bacillus* sp. strains such as *Bacillus subtilis,* and *Bacillus thuringiensis,* and various Enterobacteriaceae strains such as *Salmonella typhimurium, Serratia marcescens,* and Pseudomonas species.

When the vector is transformed into eukaryotic cells to generate recombinant microorganisms, examples of host cells that may be used include, but are not limited to, yeast (e.g., *Saccharomyces cerevisiae*)*,* insect cells, plant cells and animal cells, such as Sp2/0, CHO K1, CHO DG44, PER.C6, W138, BHK, COS7, 293, HepG2, Huh7, 3T3, RIN, and MDCK cell lines.

As used herein, the term "transformation" refers to a phenomenon in which external DNA is introduced into a host cell, thereby artificially causing genetic changes, and the term "transformant" refers to a host cell into which external DNA has been introduced and in which the expression of the gene of interest is stably maintained.

The transformation may be performed using a suitable vector introduction technique selected depending on the host cell, so that the gene of interest or a recombinant vector comprising the same may be expressed in the host cell. For example, introduction of the vector may be performed by electroporation, heat-shock, calcium phosphate (CaPO₄) precipitation, calcium chloride (CaCl₂) precipitation, microinjection, polyethylene glycol (PEG) method, DEAE-dextran method, cationic liposome method, lithium acetate-DMSO method, or a combination thereof, without being limited thereto. As long as the transformed gene may be expressed in the host cell, it may be integrated into and placed in the chromosome of the host cell, or it may exist extrachromosomally, without being limited thereto.

The transformant may include a cell transfected, transformed, or infected with the recombinant vector of the present disclosure *in vivo* or *in vitro,* and may be used in the same sense as a recombinant host cell, a recombinant cell, or a recombinant microorganism.

According to one embodiment of the present invention, the transformant may be an *Escherichia* sp. strain.

Examples of the Escherichia sp. strain include, but are not limited to, *Escherichia coli, Escherichia albertii, Escherichia blattae, Escherichia fergusonii, Escherichia hermannii, Escherichia vulneris,* and the like.

The transformant in the present invention may be a strain either comprising the above-described sigma 38 variant or a polynucleotide encoding the same or comprising the vector comprising the same, a strain expressing the sigma 38 variant or the polynucleotide, or a strain having activity for the sigma 38 variant, without being limited thereto.

According to one embodiment of the present invention, the transformant may have the ability to produce an L-aromatic amino acid.

The transformant may naturally have the ability to produce an L-aromatic amino acid or may be one artificially endowed with the ability to produce L-aromatic amino acids.

According to one embodiment of the present invention, the transformant may have an increased ability to produce an L-aromatic amino acid, due to a change in the activity of sigma 38.

As used herein, the term "increased ability to produce" means that L-aromatic amino acid productivity has increased compared to that of the parent strain. As used herein, the term "parent strain" refers to a wild-type strain or mutant strain to be mutated, and includes a strain that is to be mutated directly or to be transformed with a recombinant vector or the like. In the present invention, the parent strain may be a wild-type *Escherichia* sp. strain or an *Escherichia* sp. strain mutated from the wild-type strain.

The transformant according to the present invention exhibits an increased ability to produce an L-aromatic amino acid compared to the parent strain, due to a change in the activity of sigma 38 by introduction of the sigma 38 variant thereinto. In particular, the amount of L-aromatic amino acid produced by the transformant may be at least 10%, specifically 10 to 80% (preferably 20 to 60%) higher than that produced by the parent strain, and the transformant is capable of producing 3 to 20 g of an L-aromatic amino acid per liter of a culture of the strain.

Yet another aspect of the present invention provides a method for producing an L-aromatic amino acid, the method comprising steps of: culturing the transformant in a medium; and recovering an L-aromatic amino acid from the transformant or the medium in which the transformants has been cultured.

The culturing may be performed using a suitable medium and culture conditions known in the art, and any person skilled in the art may easily adjust and use the medium and the culture conditions. Specifically, the medium may be a liquid medium, without being limited thereto. Examples of the culturing method include, but are not limited to, batch culture, continuous culture, fed-batch culture, or a combination thereof.

According to one embodiment of the present invention, the medium should meet the requirements of a specific strain in a proper manner, and may be appropriately modified by a person skilled in the art. For the culture medium for the *Escherichia* sp. strain, reference may be made to, but not limited to, a known document (Manual of Methods for General Bacteriology, American Society for Bacteriology, Washington D.C., USA, 1981).

According to one embodiment of the present invention, the medium may contain various carbon sources, nitrogen sources, and trace element components. Examples of carbon sources that may be used include: sugars and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid. These substances may be used individually or as a mixture, without being limited thereto. Examples of nitrogen sources that may be used include peptone, yeast extract, meat extract, malt extract, corn steep liquor, soybean meal, urea, or inorganic compounds such as ammonium sulfate, ammonium chloride, ammonium phosphate, ammonium carbonate, and ammonium nitrate. The nitrogen sources may also be used individually or as a mixture, without being limited thereto. Examples of phosphorus sources that may be used include, but are not limited to, potassium dihydrogen phosphate or dipotassium hydrogen phosphate or the corresponding sodium-containing salts. In addition, the culture medium may contain, but is not limited to, metal salts such as magnesium sulfate or iron sulfate, which are required for growth. In addition, the culture medium may contain essential growth substances such as amino acids and vitamins. Moreover, suitable precursors may be used in the culture medium. The medium or individual components may be added to the culture medium batchwise or in a continuous manner by a suitable method during culturing, without being limited thereto.

According to one embodiment of the present invention, the pH of the culture medium may be adjusted by adding compounds such as ammonium hydroxide, potassium hydroxide, ammonia, phosphoric acid and sulfuric acid to the microorganism culture medium in an appropriate manner during the culturing. In addition, during the culturing, foaming may be suppressed using an anti-foaming agent such as a fatty acid polyglycol ester. Additionally, to keep the culture medium in an aerobic condition, oxygen or an oxygen-containing gas (for example, air) may be injected into the culture medium. The temperature of the culture medium may be generally 20°C to 45°C, for example, 25°C to 40°C. The culturing may be continued until a desired amount of a useful substance is produced. For example, the culturing time may be 10 hours to 160 hours.

According to one embodiment of the present invention, in the step of recovering an L-aromatic amino acid from the cultured transformant or the medium in which the transformant has been cultured, the produced L-aromatic amino acid may be collected or recovered from the medium using a suitable method known in the art depending on the culture method. Examples of a method that may be used to recover the produced L-aromatic amino acid include, but are not limited to, centrifugation, filtration, extraction, spraying, drying, evaporation, precipitation, crystallization, electrophoresis, fractional dissolution (e.g., ammonium sulfate precipitation), chromatography (e.g., ion exchange, affinity, hydrophobicity and size exclusion), and the like.

According to one embodiment of the present invention, the step of recovering an L-aromatic amino acid may be performed by centrifuging the culture medium at a low speed to remove biomass and separating the obtained supernatant through ionexchange chromatography.

According to one embodiment of the present invention, the step of recovering an L-aromatic amino acid may include a process of purifying the L-aromatic amino acid.

According to one embodiment of the present invention, the L-aromatic amino acid may be at least one selected from the group consisting of L-tryptophan, L-phenylalanine, and L-tyrosine.

### Advantageous Effects

The sigma 38 variant according to the present invention is obtained by substituting one or more amino acids in the amino acid sequence constituting sigma 38 to change protein activity, and a recombinant microorganism comprising the sigma 38 variant is capable of efficiently producing an L-aromatic amino acid.

### Brief Description of Drawings

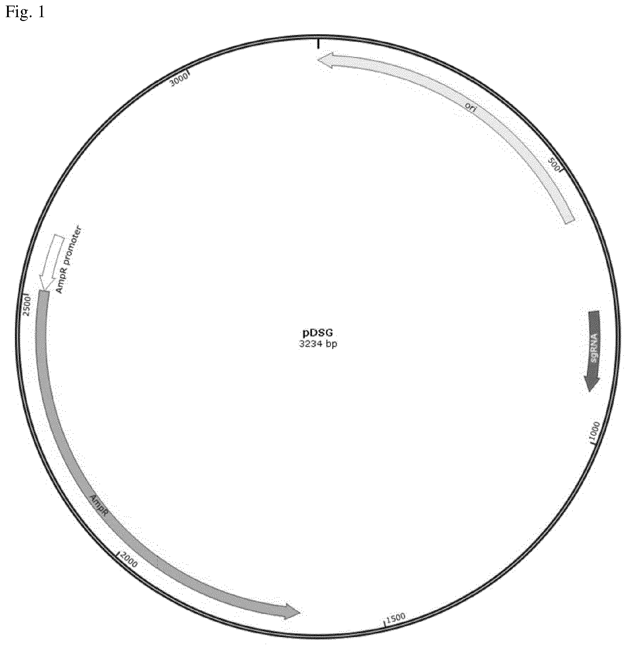

FIG. 1 shows the structure of plasmid pDSG according to an embodiment of the present invention.

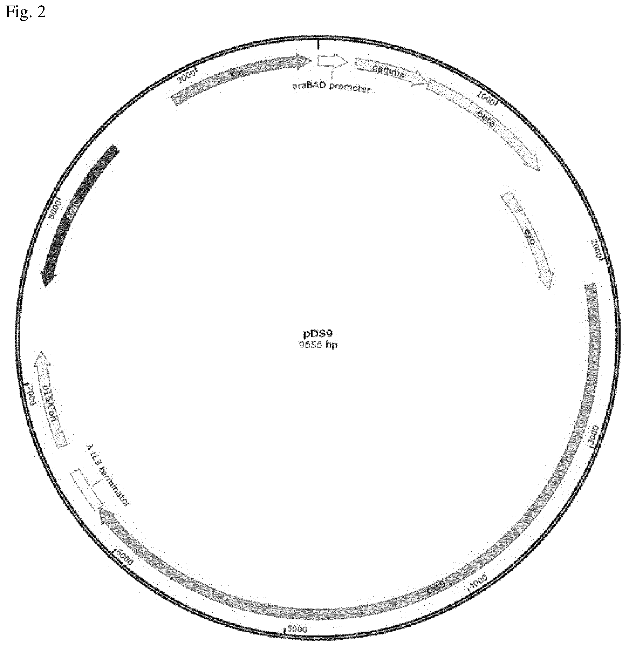

FIG. 2 shows the structure of plasmid pDS9 according to an embodiment of the present invention.

### Mode for Invention

Hereinafter, the present invention will be described in more detail. However, this description is merely presented by way of example to facilitate the understanding of the present invention, and the scope of the present invention is not limited by this exemplary description.

### Example 1. Construction of strains expressing sigma 38 variant

In order to examine the effect of a variant resulting from substitution of leucine for glutamine at position 37 of the amino acid sequence of sigma 38 (SEQ ID NO: 3) on the production of L-aromatic amino acids, a vector expressing the sigma 38 variant and strains into which the vector has been introduced were constructed. For insertion of the gene encoding the sigma 38 variant into the strains, plasmids pDSG and pDS9 were used, and the strains were constructed as follows.

Here, the plasmid pDSG has an origin of replication that works only in *Escherichia coli,* contains an ampicillin resistance gene, and has a mechanism for expressing guide RNA (gRNA). The plasmid pDS9 has an origin of replication that works only in *Escherichia coli,* contains a kanamycin resistance gene and λ red genes (exo, bet and gam), and has a mechanism for expressing CAS9 derived from *Streptococcus pyogenes.*

### 1-1. Construction of vector pDSG-rpoS(Gln37Leu) for transformation

Using *Escherichia coli* MG1655 (KCTC14419BP) gDNA as a template, a upstream fragment of the amino acid mutation at position 37 of the *E. coli* rpoS gene encoding sigma 38 was obtained by PCR using a primer pair of primer 7 and primer 9 and a primer pair of primer 8 and primer 10, and a downstream fragment of the amino acid mutation at position 37 of the *E. coli* rpoS gene was obtained by PCR using a primer pair of primer 11 and primer 13 and a primer pair of primer 12 and primer 14. At this time, each of the upstream and downstream fragments contained a sequence that modifies glutamine (Gln) at amino acid residue position 37 of the rpoS gene into leucine (Leu). In the PCR, Takara PrimeSTAR Max DNA polymerase was used as polymerase, and PCR amplification was performed for 30 cycles under the following conditions: denaturation at 95°C for 10 sec, annealing at 57°C for 15 sec, and extension at 72°C for 10 sec.

Using the plasmid pDSG as a template, four pDSG gene fragments were obtained by PCR using a primer pair of primer 3 and primer 5, a primer pair of primer 4 and primer 6, a primer pair of primer 15 and primer 1, and a primer pair of primer 16 and primer 2, respectively. At this time, each of the gene fragments contained a gRNA sequence targeting Gln of the rpoS gene. As gRNA, a 20 mer upstream of NGG of the sequence to be mutated was selected. In the PCR, Takara PrimeSTAR Max DNA polymerase was used, and PCR amplification was performed for 30 cycles under the following conditions: denaturation at 95°C for 10 sec, annealing at 57°C for 15 sec, and extension at 72°C for 15 sec.

The obtained fragments upstream and downstream of the amino acid at position 37 of the rpoS gene, and the obtained four pDSG gene fragments were cloned using a self-assembly cloning method (BioTechniques 51:55-56 (July 2011)), thereby obtaining a recombinant plasmid which was named pDSG-rpoS(Gln37Leu).

### 1-2. Construction of L-tryptophan- or L-phenylalanine-producing strain into which sigma 38 variant rpoS(Gln37Leu) has been introduced

To construct an L-tryptophan-producing strain and an L-phenylalanine-producing strain, *Escherichia coli* KCCM13013P and KCCM10016 were used as parent strains.

The KCCM13013P strain or the KCCM10016 strain was primarily transformed with the plasmid pDS9 and cultured in LB-Km (containing 25 g/L of LB liquid medium and 50 mg/L of kanamycin) solid medium, and then colonies having kanamycin resistance were selected. The selected colonies were secondarily transformed with the pDSG-rpoS(Gln37Leu) plasmid and cultured in LB-Amp & Km (containing 25 g/L of LB liquid medium, 100 mg/L of ampicillin and 50 mg/L of kanamycin) solid medium, and then ampicillin- and kanamycin-resistant colonies were selected. Next, a gene fragment was obtained by PCR using a primer pair of primer 17 and primer 18. Here, Takara PrimeSTAR Max DNA polymerase was used as polymerase, and PCR amplification was performed for 30 cycles under the following conditions: denaturation at 95°C for 10 sec, annealing at 57°C for 10 sec, and extension at 72°C for 15 sec. The sequence of the gene fragment obtained using the primer pair of primer 17 and primer 18 was analyzed by Macrogen.

The selected secondary transformants were passaged 7 times in LB liquid medium, and colonies were selected on LB solid medium. Each colony was selectively cultured on each of LB, LB-Amp and LB-Km solid media. Colonies that did not grow on the LB-Amp and LB-Km solid media while growing on the LB solid medium were selected. The strains constructed in this way were named KCCM13013P_rpoS(Gln37Leu) and KCCM10016_rpoS(Gln37Leu), respectively.

The primer sequences used in Example 1 are shown in Table 1 below.

**[Table 1]**

| Primer name | SEQ ID NO | Primer sequence (5' → 3') |
|---|---|---|
| Primer 1 | 5 | CAATTTTATTATAGTAATTGACTATTATAC |
| Primer 2 | 6 | GGAACCCAGTCAATTTTATTATAGTAATTGACTATTATAC |
| Primer 3 | 7 | ACTGGGTTCCTGTTCTACTAGTTTTAGAGCTAGAAATAGC |
| Primer 4 | 8 | TGTTCTACTAGTTTTAGAGCTAGAAATAGC |
| Primer 5 | 9 | GAGCCTGTCGGCCTACCTGCT |
| Primer 6 | 10 | CGGCCGGCATGAGCCTGTCG |
| Primer 7 | 11 | ATGCCGGCCGGGTCCGGGAACAACAAGAAG |
| Primer 8 | 12 | GGTCCGGGAACAACAAGAAG |
| Primer 9 | 13 | GCCTTTTCGTCAAAAACCTC |
| Primer 10 | 14 | TTCTACTAAGGCCTTTTCGTCAAAAACCTC |
| Primer 11 | 15 | CTTAGTAGAATTGGAACCCAGTGATAACGA |
| Primer 12 | 16 | TTGGAACCCAGTGATAACGA |
| Primer 13 | 17 | CGGATCAGCCCCAGGTTGCC |
| Primer 14 | 18 | CTCTACCGCGCGGATCAGCC |
| Primer 15 | 19 | CGCGGTAGAGGAGCTCCTGAAAATCTCGATAAC |
| Primer 16 | 20 | GAGCTCCTGAAAATCTCGATAAC |
| Primer 17 | 21 | GGCCGCGTTGTTTATGCTGG |
| Primer 18 | 22 | CTGGTGCGTATGGGCGGTAA |

### Experimental Example 1. Evaluation of L-aromatic amino acid productivity of mutant strain into which sigma 38 variant has been introduced

The L-tryptophan or L-phenylalanine productivities of the parent strains (KCCM13013P and KCCM10016) and the mutant strains (KCCM13013P_rpoS(Gln37Leu) and KCCM10016_rpoS(Gln37Leu)) into which the sigma 38 variant has been introduced were compared.

1 vol% of each strain (parent strain or mutant strain) was inoculated into a 100-mL flask containing 10 mL of a medium for tryptophan production or a medium for phenylalanine production (the composition of each medium is shown in Table 2 below) and was cultured with shaking at 37°C and 200 rpm for 72 hours. After completion of the culturing, the concentration of L-tryptophan or L-phenylalanine in the medium was measured using HPLC (Agilent), and the results are shown in Tables 3 and 4, respectively.

**[Table 2]**

| Medium for tryptophan production | | Medium for phenylalanine production | |
|---|---|---|---|
| Component | Content | Component | Content |
| Glucose | 80.0 g/L | Glucose | 80.0 g/L |
| (NH₄)₂SO₄ | 20.0 g/L | (NH₄)₂SO₄ | 20.0 g/L |
| K₂HPO₄ | 0.8 g/L | K₂HPO₄ | 1.0 g/L |
| K₂SO₄ | 0.4 g/L | KH₂PO₄ | 1.0 g/L |
| MgCl₂ | 0.8 g/L | K₂SO₄ | 0.4 g/L |
| Fumaric acid | 1.0 g/L | MgCl₂ | 1.0 g/L |
| Yeast extract | 1.0 g/L | Fumaric acid | 0.5 g/L |
| (NH₄)₆Mo₇O₂₄ | 0.12 ppm | Yeast extract | 1.0 g/L |
| H₃BO₃ | 0.01 ppm | Glutamic acid | 0.5 g/L |
| CuSO₄ | 0.01 ppm | CaCl₂ | 5.00 ppm |
| MnCl₂ | 2.00 ppm | MnCl₂ | 2.00 ppm |
| ZnSO₄ | 0.01 ppm | ZnSO₄ | 1.00 ppm |
| CoCl₂ | 0.10 ppm | CoCl₂ | 0.10 ppm |
| FeCl₂ | 10.00 ppm | FeCl₂ | 10.00 ppm |
| Thiamine_HCl | 20.00 ppm | Thiamine_HCl | 20.00 ppm |
| L-Tyrosine | 200.00 ppm | L-Tyrosine | 200.00 ppm |
| L-phenylalanine | 300.00 ppm | CaCO₃ | 3% |
| CaCO₃ | 3% | - | - |
| pH 7.0 with NaOH (33%) | | pH 7.0 with NaOH (33%) | |

**[Table 3]**

| Strain | L-tryptophan concentration (g/L) | L-tryptophan concentration increase rate (%) |
|---|---|---|
| KCCM13013P | 4.02 | - |
| KCCM13013P_rpoS(Gln37Leu) | 5.00 | 24.3 |

**[Table 4]**

| Strain | L-phenylalanine concentration (g/L) | L-phenylalanine concentration increase rate (%) |
|---|---|---|
| KCCM10016 | 3.01 | - |
| KCCM10016_rpoS(Gln37Leu) | 3.74 | 24.2 |

As shown in Tables 3 and 4 above, it was confirmed that the mutant strains KCCM13013P_rpoS(Gln37Leu) and KCCM10016_rpoS(Gln37Leu) into which the sigma 38 variant has been introduced showed about 24% increases in L-tryptophan and L-phenylalanine production compared to the parent strains KCCM13013P and KCCM10016.

So far, the present invention has been described with reference to the embodiments. Those skilled in the art will appreciate that the present invention can be implemented in modified forms without departing from the essential features of the present invention. Therefore, the disclosed embodiments should be considered in descriptive sense only and not for purposes of limitation. Therefore, the scope of the present invention is defined not by the detailed description of the present invention but by the appended claims, and all differences within a range equivalent to the scope of the appended claims should be construed as being included in the present invention.

## Claims

1. A sigma 38 variant composed of the amino acid sequence of SEQ ID NO: 1 in which glutamine at position 37 in the amino acid sequence of SEQ ID NO: 3 is substituted with leucine.

2. A polynucleotide encoding the variant of claim 1.

3. A transformant comprising the variant of claim 1 or the polynucleotide of claim 2.

4. The transformant of claim 3, which is an *Escherichia* sp. strain.

5. The transformant of claim 3, which has ability to produce an L-aromatic amino acid.

6. A method for producing an L-aromatic amino acid, the method comprising steps of:
culturing the transformant of claim 3 in a medium; and
recovering an L-aromatic amino acid from the transformant or the medium in which the transformant has been cultured.

7. The method of claim 6, wherein the L-aromatic amino acid is at least one selected from the group consisting of L-tryptophan, L-phenylalanine, and L-tyrosine.
